# EUROPEAN PATENT SPECIFICATION

(11) **EP 3 965 886 B1**
(45) Date of publication and mention of the grant of the patent: **23.04.2025**
(21) Application number: 20754134.3
(22) Date of filing: 14.02.2020
(51) Int. Cl.: A61P 11/02, A61K 9/00, A61K 9/12, A61P 11/06, A61K 31/135, A61K 31/137, A61K 31/047, A61K 31/58

(54) **NASAL SPRAY COMPOSITIONS AND RELATED TREATMENT METHODS**
NASENSPRAYZUSAMMENSETZUNGEN UND VERWANDTE BEHANDLUNGSVERFAHREN
COMPOSITIONS DE PULVÉRISATION NASALE ET PROCÉDÉS DE TRAITEMENT ASSOCIÉS

(30) Priority: 15.02.2019 US 201962806364 P
(43) Date of publication of application: 16.03.2022
(73) Proprietor: Ferrer Medical Innovations, LLC, Aventura, Florida 33180 (US)
(72) Inventor: FERRER, Gustavo, Aventura, Florida 33180 (US)
(74) Representative: Elósegui de la Peña, Iñigo
(86) International application number: PCT/US2020/018430
(87) International publication number: WO 2020/168289

(56) References cited:
- WO-A1-97/46243
- US-A- 5 288 492
- US-A1- 2006 216 353
- US-A1- 2008 058 421
- US-B1- 6 319 513
- US-B2- 6 793 942

## Description

### SUMMARY

Disclosed herein are various compositions that may be used to treat various conditions including cold and allergy symptoms such as nasal drip, coughing, sneezing, sinusitis, and the like. Some embodiments and implementations may also provide an antiviral effect and/or be preventative of viral disease, either using chlorpheniramine individually or in combination with xylitol. In preferred embodiments and implementations of related methods, the composition may be provided in the form of a nasal spray and/or be delivered into a subject's nasal and/or sinus passages. However, it is anticipated that alternative embodiments and implementations may be made into suspensions, lozenges, tablets, capsules, topical formulations, and/or ingestible products, such as teas or other beverages, for example, or other liquid formulations that may be inserted into nasal passages other than in the form of a spray, such as drops.

For treating a cold or allergy condition and restoring nasal flora in a human, a nasal spray composition comprises chlorpheniramine in a concentration of between about 0.4% and about 10% by mass (in more preferred compositions, between about 0.4% and about 1 % by mass); xylitol in a concentration of between about 10% and about 15% by mass; and fluticasone propionate in a concentration of between about 0.03% and about 1.0% by mass. The nasal spray composition may then be delivered into the human subject's nasal passages.

In some implementations, the nasal spray composition further comprises aloe vera. In some such implementations, the aloe vera is present in the composition in a concentration of between about 0.02% and about 2%.

In some implementations, the nasal spray composition further comprises at least one of benzalkonium chloride and grapefruit seed extract.

In some implementations, the step of delivering the nasal spray composition may comprise delivering 1-2 sprays of the composition in each nostril every six, eight, or twelve hours.

In another specific example the composition for the treatment of a cold or allergy condition in a human according to other implementations, comprises an antihistamine comprising at least one of chlorpheniramine and diphenhydramine. The antihistamine is present in a concentration of between about 0.25% and about 10% by mass (in more preferred formulations, between about 0.25% and about 4% by mass). The composition may further comprise a corticosteroid, which may be present in a concentration of between about 0.01 % and about 3% by mass. The composition may further comprise xylitol in a concentration of between about 5% and about 15% by mass. The composition may be delivered into the human subject's nasal passages.

In some implementations, the corticosteroid is fluticasone propionate. In some such implementations, the fluticasone propionate is present in the composition in a concentration of between about 0.03% and about 1 % by mass.

In some implementations, the xylitol is present in the composition in a concentration of between about 10% and about 15% by mass.

In some implementations, the nasal spray composition further comprises aloe vera, which, in some such implementations, is present in the composition in a concentration of between about 0.02% and about 2%.

In an example of a composition, such as a nasal spray composition for use in the treatment of a cold or allergy condition, the composition comprises an antihistamine, such as chlorpheniramine and/or diphenhydramine, which is present in the composition in a concentration of between about 0.25% and about 10% (in more preferred compositions, between about 0.25% and about 4%) by mass. The composition further comprises xylitol.

The xylitol is present in the composition in a concentration of between about 5% and about 15% by mass. In some embodiments, the xylitol is present in the composition in a concentration of between about 10% and about 15% by mass. The composition may further comprise the corticosteroid fluticasone propionate, which is present in the composition in a concentration of between about 0.01 % and about 3% by mass.

In some embodiments, fluticasone propionate is present in the composition in a concentration of between about 0.03% and about 1.0% by mass.

In some embodiments, the antihistamine comprises chlorpheniramine. In some such embodiments, the chlorpheniramine is present in the composition in a concentration of between about 0.4% and about 10% by mass (in more preferred compositions, between about 0.4 and about 1.0% by mass).

Some embodiments may further comprise aloe vera, which preferably is present in the composition in a concentration of between about 0.02% and about 3.0% by mass. In some such embodiments, the aloe vera is present in the composition in a concentration of between about 0.05% and about 2.0% by mass.

The features, structures, steps, or characteristics disclosed herein in connection with one embodiment or implementation may be combined in any suitable manner in one or more alternative embodiments or implementations. It should also be understood that any reference to a detail associated with an "embodiment" may be incorporated into and/or used with an "implementation," and *vice versa.*

### DETAILED DESCRIPTION

Disclosed herein are various compositions that may be provided in nasal sprays and/or otherwise delivered into a subject's nasal and/or sinus passages. Preferred embodiments of such compositions, and preferred implementations of related methods, may be used to treat various conditions including cold and allergy symptoms such as nasal drip, coughing, sneezing, sinusitis, and the like. Some embodiments may also allow for restoring and/or improving the natural flora of the nasal cavity to provide more long-lasting health improvements. However, it is anticipated that alternative embodiments and implementations may be made into suspensions, lozenges, tablets, capsules, topical formulations, and/or ingestible products, such as teas or other beverages, for example.

Research has indicated that use of a nasal saline alone is insufficient for the treatment of various symptoms associated with allergies and chronic sinusitis and/or rhinitis. Other treatment options and/or protocols have been tried, such as a protocol that combines saline and three other nasal spray formulations. This protocol involves using the nasal saline twice daily to cleanse the nasal passages and prepare them for delivery of the other sprays.

The three formulations involved in this protocol include one that comprises an agent used for blocking mucus production, such as azelastine hydrochloride, one that comprises an agent used for nasal drying, such as ipratropium bromide, and one that comprises a corticosteroid for decreasing inflammation, such as flunisolide. According to the protocol, these formulations are to be delivered with two sprays into each nostril twice daily for one month, after which the symptoms are reevaluated. If the symptoms have improved, the same regimen is continued for an additional two months, after which the dosage may be decreased to a single spray in each nostril twice daily, and then after an additional month to two months may be decreased again to a single spray in each nostril once daily.

Although effective, this protocol suffers from a number of drawbacks and disadvantages. For example, it is rather cumbersome and requires a patient to obtain and/or mix several different ingredients and/or formulations. This can also lead to confusion, misinterpretation, and/or misuse of the protocol guidelines, which can cause poor performance, overdose, and increase the risk of unwanted side effects. In addition, several of the formulations and/or ingredients are unpleasant in taste and/or smell and can cause an undesirable aftertaste (azelastine hydrochloride), also limiting its usefulness in treating children. The protocol is also rather expensive due to the various formulations involved. The protocol also has room for improvement in terms of efficacy. For example, the side effects can lead to dry nasal tissue, bacterial infections, and other side effects. Preferably, the formulation will be available over the counter and will provide for treatment of sinusitis, rhinitis, and otitis in a single composition that may replace, and improve upon, a full regimen of other nasal sprays.
US5288492A, Decongestant composition containing aloe vera, provides a decongestant composition in the form of an aqueous solution comprising aloe vera, castor oil, sodium metasilicate and calcium chloride as the essential active components. The combination of specific active components are formulated in such relative proportions as to bring about a decongestant effect, whereby topical administration of the compositions to the nasal or bronchial passage according the method of the present invention produces a marked therapeutic result, as well as a moisturizing effect on breathing passages.

The preferred compositions for the use disclosed herein overcome many of these drawbacks and/or other drawbacks of the prior art by, in some preferred embodiments and/or disclosed treatment methods, combining the most desirable agents and concentrations into a single composition, preferably in the form of a nasal spray, and may include other ingredients for decreasing various side effects, such as xylitol or another non-hexose sugar alcohol, which may provide a number of benefits, such as inhibiting bacterial growth, fungal growth, decreasing undue nasal drying, enhancing antiinflammatory benefits, and/or decreasing the unwanted aftertaste of the product.

In most preferred compositions, an antihistamine is provided, such as, in preferred embodiments, chlorpheniramine and/or diphenhydramine. The chlorpheniramine or another suitable antihistamine is present in the formulation in a concentration between about 0.25 and about 10% by mass (more preferably, between about 0.25 and about 4% by mass). In more preferred embodiments, the chlorpheniramine and/or diphenhydramine is present in the formulation in a concentration between about 2% and about 3% by mass.

In certain embodiments of formulations comprising diphenhydramine, the concentration may be slightly less. For example, in some such embodiments, the concentration of diphenhydramine may be between about 1 % and about 2% by mass.

Preferably, corticosteroids are included in the formulation. For example, in preferred embodiments, fluticasone propionate, is included. The corticosteroid may be present in the formulation in a concentration between about 0.01 and about 3% by mass, and comprises fluticasone propionate. In some such formulations, the fluticasone propionate is present in a concentration of between about 0.03% and about 1 % by mass. In more preferred formulations, the fluticasone propionate is present in a concentration of about 0.05% by mass.

In most preferred compositions, xylitol is included in the formulation. Including xylitol may provide a number of benefits and may provide a synergistic effect with the other ingredients of the formulation. Xylitol in particular has been shown to be very effective in moisturizing nasal passages and the like. Without being limited by theory, this is thought to occur because xylitol can create a hyper-osmotic solution that pulls moisture towards it from surrounding tissues without generated mucous. Thus, the combination of xylitol, may result in a decrease in mucous production, potentially along with accompanying anti-bacterial and other health benefits associated with xylitol .

Xylitol may also enhance the taste and/or reduce the negative smell/aftertaste issues commonly associated with prior art formulations. Moreover, by providing a sweetener that does not include sugar and serves as an active agent in the formulation, several benefits may be derived. For example, although xylitol acts as a sweetener, unlike typical sweeteners, xylitol enhances the ability of other agents to treat bacterial infections by actively starving the microorganisms causing the symptoms, rather than one that counteracts the active ingredients in a nasal spray or other treatment composition by feeding the microorganisms, or one that only passively starves the microorganisms by providing a sweetener that is not consumed by the more common oral and pharyngeal pathogens. Thus, compositions including xylitol may result in improved ability to treat various symptoms that may be caused by microorganisms (along with other not caused by microorganisms) and, more importantly, to fight the bacterial and/or other microorganisms behind these symptoms.

Preferably, xylitol is present in a concentration of between about 5 and about 15% by mass. In more preferred formulations, xylitol is present in a concentration of between about 10 and about 15% by mass. Although xylitol has been used as a sweetener in a variety of products, it should be noted that it is considered an active ingredient in the preferred formulations for use of the invention disclosed herein due to its moisturizing and/or antibacterial properties.

More preferred formulations may include various other ingredients, such as aloe vera. Including aloe vera, preferably along with xylitol, may enhance the synergistic effects referenced elsewhere herein. More particularly, aloe vera may provide a soothing, moisturizing, and/or cooling effect that may increase user compliance and/or may heal irritated tissue, such as nasal and/or sinus passages in preferred embodiments, which may be caused by, for example, repeated nose wiping, sneezing, nose blowing, and the like. In preferred formulations, aloe vera is present in a concentration of between about 0.05 and about 3.0% by mass.

These formulations include grapefruit see extract, benzalkonium chloride preferably along with purified water. Thus, in some such embodiments, grapefruit seed extract is included in the formulation in an amount between about 0.01 and about 3% by mass.

In embodiments comprising benzalkonium chloride, which may be a suitable substitute for grapefruit seed extract, is present in a concentration of between about 0.05 and about 0.2% by mass. The remainder of the formulation may comprise (preferably purified) water and glycerine. In some formulations, purified water may be present in a range from about 80 to about 98% by mass. In some such formulations, the purified water may be present in a range from about 80 to about 90% by mass. In a more preferred formulation, water may be present in a concentration of about 87.5%. Glycerine, if included, preferably ranges between about 0.05 and about 3% by mass.

Additional more specific and, generally speaking, more preferred, embodiments of the invention are disclosed below. Although these formulations for use have very specific ingredients and concentrations, it should be understood that the concentrations of the ingredients in these formulations may vary by around 5% from the concentrations provided herein.

### Example 1

In a more particular example of a composition, preferably in the form of a nasal spray, for use in the treatment of various symptoms associated with allergies, sinusitis, colds, and the like, the composition may comprise the following ingredients in at least approximately the concentrations (by mass) presented in the chart below. However, as mentioned above, these concentrations may vary by, for example, by about 5% in certain alternative embodiments and related methods. Similarly, one or more of these ingredients may be omitted and/or replaced with an alternative substantially similar ingredient in still other embodiments and related methods.

| | |
|---|---|
| Diphenhydramine | 1 to 2% |
| Fluticasone propionate | 0.03 to 1% |
| Xylitol | 10 to 15% |
| Aloe Vera | 0.02 to 2% |
| Benzalkonium chloride | 0.02 to 0.05% |
| Glycerine | 1 to 2% |
| Purified Water | 80 to 90% |

This composition for use according to claims is delivered into a subject's nasal cavity every twelve hours, preferably in the form of 1-2 sprays in each nostril. The present inventor has found that doing so is highly effective in drying the nasal mucosa, reducing inflammation, and/or restoring the natural, nasal flora. It can therefore be used to treat, for example, acute or chronic sinusitis and related conditions, including nasal drip, cough, and nasal dryness.

### Example 2

In a more particular example of a composition for use according to claims, preferably in the form of a nasal spray, for treating various symptoms associated with allergies, sinusitis, colds, and the like, the composition comprises the following ingredients in at least approximately the concentrations (by mass) presented in the chart below. However, as mentioned above, these concentrations may vary by, for example, by about 5% in certain alternative embodiments and related methods. Similarly, one or more of these ingredients may be omitted and/or replaced with an alternative substantially similar ingredient in still other embodiments and related methods.

| | |
|---|---|
| Diphenhydramine | 1% |
| Mometasone | 0.05% |
| Xylitol | 11% |
| Aloe Vera | 0.25% |
| Grapefruit Seed Extract | 0.2% |
| Glycerine | 1% |
| Purified Water | 87.5% |

This composition for use according to claims is delivered into a subject's nasal cavity every twelve hours, preferably in the form of 1- 2 sprays in each nostril. The present inventor has found that doing so is highly effective in drying the nasal mucosa, reducing inflammation, and/or restoring the natural, nasal flora. It can therefore be used to treat, for example, acute or chronic sinusitis and related conditions, including nasal drip, cough, and nasal dryness.

### Example 3

In another more particular example of a composition, preferably in the form of a nasal spray, for use in the treatment of various symptoms associated with allergies, sinusitis, colds, and the like, the composition for use according to claims comprises the following ingredients in at least approximately the concentrations (by mass) presented in the chart below. However, as mentioned above, these concentrations may vary by 5% in certain alternative embodiments and related methods. Similarly, one or more of these ingredients may be omitted and/or replaced with an alternative substantially similar ingredient in still other embodiments and related methods.

| | |
|---|---|
| Chlorpheniramine | 0.4% |
| Mometasone | 0.05% |
| Xylitol | 11% |
| Aloe Vera | 0.25% |
| Grapefruit Seed Extract | 0.2% |
| Glycerine | 1% |
| Purified Water | 87.5% |

Because this is a more potent formulation than that of Example 1, the composition is delivered into a subject's nasal cavity every four to six hours, preferably in the form of 1-2 sprays in each nostril. The present inventor has found that doing so is highly effective in drying the nasal mucosa, reducing inflammation, and/or restoring the natural, nasal flora. It can therefore be used to treat, for example, acute or chronic sinusitis and related conditions, including nasal drip, cough, and nasal dryness.

### Example 4

In another more particular example of a composition, preferably in the form of a nasal spray, for use in the treatment of treating various symptoms associated with allergies, sinusitis, colds, and the like, the composition for use according to claims comprises the following ingredients in at least approximately the concentrations (by mass) presented in the chart below. However, as mentioned above, these concentrations may vary by 5% in certain alternative embodiments and related methods. Similarly, one or more of these ingredients may be omitted and/or replaced with an alternative substantially similar ingredient in still other embodiments and related methods.

| | |
|---|---|
| Chlorpheniramine | 0.4 to 10% (more preferably 0.4 to 1.0%) |
| Xylitol | 10 to 15% |
| Aloe Vera | 0.05 to 2% |
| Benzalkonium Chloride | 0.02 to 0.05% |
| Glycerine | 1 to 2% |
| Purified Water | 80 to 90% |

Because this is a more potent formulation than that of Example 1, the composition is delivered into a subject's nasal cavity every four to six hours, preferably in the form of 1-2 sprays in each nostril. The present inventor has found that doing so is highly effective in drying the nasal mucosa, reducing inflammation, and/or restoring the natural, nasal flora. It can therefore be used to treat, for example, acute or chronic sinusitis and related conditions, including nasal drip, cough, and nasal dryness.

### Example 5

In another more particular example of a composition, preferably in the form of a nasal spray, for use in the treatment of various symptoms associated with allergies, sinusitis, colds, and the like, the composition for use according to claims comprises the following ingredients in at least approximately the concentrations (by mass) presented in the chart below.

| | |
|---|---|
| Diphenhydramine | 1 to 2% |
| Xylitol | 10 to 15% |
| Aloe Vera | 0.05 to 2% |
| Benzalkonium Chloride | 0.02 to 0.05% |
| Glycerine | 1 to 2% |
| Purified Water | 80 to 90% |

The composition is delivered into a subject's nasal cavity every twelve hours, preferably in the form of 1-2 sprays in each nostril. Thepresent inventor has found that doing so is highly effective in drying the nasal mucosa and/or restoring the natural, nasal flora. It can therefore be used to treat, for example, allergy and/or cold symptoms, including nasal drip, cough, and nasal dryness.

### Example 6

In yet another more particular example of a composition, preferably in the form of a nasal spray, for use in the treatment of various symptoms associated with allergies, sinusitis, colds, and the like, the composition comprises the following ingredients in at least approximately the concentrations (by mass) presented in the chart below.

| | |
|---|---|
| Diphenhydramine | 1% |
| Xylitol | 11% |
| Aloe Vera | 0.25% |
| Grapefruit Seed Extract | 0.2% |
| Glycerine | 1% |
| Purified Water | 87.5% |

The composition is delivered into a subject's nasal cavity every four to six hours, preferably in the form of 1-2 sprays in each nostril. The present inventor has found that doing so is highly effective in drying the nasal mucosa and/or restoring the natural, nasal flora. It can therefore be used to treat, for example, allergy and/or cold symptoms, including nasal drip, cough, and nasal dryness.

### Example 7

In yet another even more particular example of a composition, preferably in the form of a nasal spray, for use in the treatment of various symptoms associated with allergies, sinusitis, colds, and the like, the composition comprises the following ingredients in at least approximately the concentrations (by mass) presented in the chart below.

| | |
|---|---|
| Chlorpheniramine | 0.4% to 10% (in more particular compositions, 0.4%) |
| Fluticasone propionate | 0.03 to 1% |
| Xylitol | 10 to 15% |
| Aloe Vera | 0.02 to 2% |
| Benzalkonium chloride | 0.02 to 0.05% |
| Glycerine | 1 to 2% |
| Purified Water | 80 to 90% |

### Example 8

In another example of a composition according to other embodiments, preferably in the form of a nasal spray, for use in the treartment of various symptoms associated with allergies, sinusitis, colds, and the like, the composition may comprise the following ingredients in at least approximately the concentrations (by mass) presented in the chart below.

| | |
|---|---|
| Chlorpheniramine | 0.4 to 10.0% |
| Fluticasone propionate | 0.03 to 1% |
| Aloe Vera | 0.02 to 2% |
| Benzalkonium chloride | 0.02 to 0.05% |
| Glycerine | 1 to 2% |
| Purified Water | 80 to 90% |

### Example 9

In still another example of a composition according to other embodiments, preferably in the form of a nasal spray, for use in the treatment of various symptoms associated with allergies, sinusitis, colds, and the like, the composition may comprise the following ingredients in at least approximately the concentrations (by mass) presented in the chart below.

| | |
|---|---|
| Chlorpheniramine | 0.4 to 10.0% |
| Aloe Vera | 0.02 to 2% |
| Benzalkonium chloride | 0.02 to 0.05% |
| Glycerine | 1 to 2% |
| Purified Water | 80 to 90% |

It will be understood by those having skill in the art that changes may be made to the details of the above-described embodiments without departing from the underlying principles presented herein. For example, the compositions disclosed herein may be administered via liquid drops from a dropper, topically (in some cases using a cotton swab or the like), orally, via a mister or atomizer, and/or via any other suitable manner of administration. In addition, alternative compositions and treatment according to the disclosure are contemplated in which the preferred nasal sprays may be replaced with suspensions, lozenges, tablets, capsules, topical formulations, and/or ingestible products, such as teas or other beverages, for example. In addition, any suitable combination of various embodiments, or the features thereof, is contemplated.

Throughout this specification, any reference to "one embodiment," "an embodiment," or "the embodiment" means that a particular feature, structure, or characteristic described in connection with that embodiment is included in at least one embodiment. Thus, the quoted phrases, or variations thereof, as recited throughout this specification are not necessarily all referring to the same embodiment.

Similarly, it should be appreciated that in the above description of embodiments, various features are sometimes grouped together in a single embodiment, figure, or description thereof for the purpose of streamlining the disclosure. This method of disclosure, however, is not to be interpreted as reflecting an intention that any claim require more features than those expressly recited in that claim. Rather, inventive aspects lie in a combination of fewer than all features of any single foregoing disclosed embodiment. It will be apparent to those having skill in the art that changes may be made to the details of the above-described embodiments without departing from the underlying principles set forth herein.

Likewise, benefits, other advantages, and solutions to problems have been described above with regard to various embodiments. However, benefits, advantages, solutions to problems, and any element(s) that may cause any benefit, advantage, or solution to occur or become more pronounced are not to be construed as a critical, a required, or an essential feature or element. The scope of the present invention should, therefore, be determined only by the following claims.

## Claims

1. Composition comprising:
chlorpheniramine in a concentration of between about 0.4% and about 1% by mass;
xylitol in a concentration of between about 10% and about 15% by mass;
fluticasone propionate in a concentration of between about 0.03% and about 1.0% by mass;
aloe vera in a concentration of between about 0.02% and about 2%; and
at least one of the benzalkonium chloride and grapefruit seed extract;
for use in a method for the treatment of cold, influenza, viral infection, rhinitis, sinusitis, or allergy condition, and restoring nasal flora in a human, wherein the composition is administered as a nasal spray composition into the human subject's nasal passages, delivering 1-2 sprays of the composition in each nostril every six, eight, or twelve hours.

2. Composition for use in a method for the treatment of cold, influenza, viral infection, rhinitis, sinusitis, or allergy condition in a human, comprising:
an antihistamine comprising at least one of chlorpheniramine and diphenhydramine, the antihistamine comprising a concentration of between about 0.25% and about 10% by mass;
fluticasone -propionate in a concentration of between about 0.03% and about 1% by mass;
xylitol in a concentration of between about 10% and about 15% by mass; and
aloe Vera in a concentration of between about 0.02% and about 2%.

3. A nasal spray composition for treatment of a cold or allergy condition, the composition comprising:
an antihistamine comprising at least one of chlorpheniramine and diphenhydramine, the antihistamine comprising a concentration of between about 0.25% and about 4% by mass;
xylitol in a concentration of between about 10% and about 15% by mass;
fluticasone propionate in a concentration of between about 0.03% and about 1.0% by mass; and
aloe Vera in a concentration of between about 0.02% and about 3.0% by mass.

4. The nasal spray composition of claim **3,** wherein the antihistamine comprises chlorpheniramine.

5. The nasal spray composition of claim **4,** wherein the chlorpheniramine is present in the composition in a concentration of between about 0.4% and about 1.0% by mass.

6. The nasal spray composition of claim **3,** wherein the aloe Vera is present in the composition in a concentration of between about 0.05% and about 2.0% by mass.

## Patentansprüche

1. Zusammensetzung, umfassend:
Chlorpheniramin in einer Konzentration zwischen etwa 0,4 und etwa 1 Massenprozent;
Xylit in einer Konzentration zwischen etwa 10 und etwa 15 Massenprozent;
Fluticasonpropionat in einer Konzentration zwischen etwa 0,03 % und etwa 1,0 Massenprozent;
Aloe vera in einer Konzentration zwischen etwa 0,02 % und etwa 2 %; und
mindestens eines von Benzalkoniumchlorid und Grapefruitkernextrakt;
zur Verwendung in einem Verfahren zur Behandlung einer Erkältung, Grippe, Virusinfektion, Rhinitis, Sinusitis oder eines Allergiezustands und zur Wiederherstellung der Nasenflora bei einem Menschen, wobei die Zusammensetzung als Nasensprayzusammensetzung in die Nasengänge des Menschen verabreicht wird, wobei alle sechs, acht oder zwölf Stunden 1-2 Sprühstöße der Zusammensetzung in jedes Nasenloch abgegeben werden.

2. Zusammensetzung zur Verwendung in einem Verfahren zur Behandlung einer Erkältung, Grippe, Virusinfektion, Rhinitis, Sinusitis oder eines Allergiezustands bei einem Menschen, umfassend:
ein Antihistaminikum, das mindestens eines von Chlorpheniramin und Diphenhydramin umfasst, wobei das Antihistaminikum eine Konzentration zwischen etwa 0,25 und etwa 10 Massenprozent umfasst;
Fluticasonpropionat in einer Konzentration zwischen etwa 0,03 % und etwa 1 Masseprozent;
Xylit in einer Konzentration zwischen etwa 10 und etwa 15 Massenprozent; und
Aloe vera in einer Konzentration zwischen etwa 0,02 % und etwa 2 %.

3. Nasensprayzusammensetzung zur Behandlung eines Erkältungs- oder Allergiezustands, wobei die Zusammensetzung umfasst:
ein Antihistaminikum, das mindestens eines von Chlorpheniramin und Diphenhydramin umfasst, wobei das Antihistaminikum eine Konzentration zwischen etwa 0,25 und etwa 4 Massenprozent umfasst;
Xylit in einer Konzentration zwischen etwa 10 und etwa 15 Massenprozent;
Fluticasonpropionat in einer Konzentration zwischen etwa 0,03 % und etwa 1,0 Massenprozent; und
Aloe vera in einer Konzentration zwischen etwa 0,02 % und etwa 3,0 % Massenprozent.

4. Nasensprayzusammensetzung nach Anspruch **3,** wobei das Antihistaminikum Chlorpheniramin umfasst.

5. Nasensprayzusammensetzung nach Anspruch **4,** wobei das Chlorpheniramin in der Zusammensetzung in einer Konzentration zwischen etwa 0,4 und etwa 1,0 Massenprozent vorhanden ist.

6. Nasensprayzusammensetzung nach Anspruch **3,** wobei die Aloe vera in der Zusammensetzung in einer Konzentration zwischen etwa 0,05 und etwa 2,0 Massenprozent vorhanden ist.

## Revendications

1. Composition comprenant :
de la chlorphéniramine à une concentration entre environ 0,4 % et environ 1 % en masse ;
du xylitol à une concentration entre environ 10 % et environ 15 % en masse ;
du propionate de fluticasone à une concentration entre environ 0,03 % et environ 1,0 % en masse ;
de l'aloe vera à une concentration entre environ 0,02 % et environ 2 % ; et
au moins l'un parmi le chlorure de benzalkonium et l'extrait de pépins de pamplemousse ;
destinée à être utilisée dans un procédé pour le traitement du rhume, de la grippe, de l'infection virale, de la rhinite, de la sinusite ou de l'allergie, et la restauration de la flore nasale chez l'homme, dans laquelle la composition est administrée sous la forme d'une composition de vaporisateur nasal dans les voies nasales du sujet humain, délivrant 1 à 2 pulvérisations de la composition dans chaque narine toutes les six, huit ou douze heures.

2. Composition destinée à être utilisée dans un procédé pour le traitement du rhume, de la grippe, de l'infection virale, de la rhinite, de la sinusite ou de l'allergie chez l'homme, comprenant :
un antihistaminique comprenant au moins l'une parmi la chlorphéniramine et la diphénhydramine, l'antihistaminique comprenant une concentration entre environ 0,25 % et environ 10 % en masse ;
le propionate de fluticasone dans une concentration entre environ 0,03 % et environ 1 % en masse ;
du xylitol à une concentration entre environ 10 % et environ 15 % en masse ; et
de l'aloe vera à une concentration entre environ 0,02 % et environ 2 %.

3. Composition de pulvérisation nasale pour le traitement d'un rhume ou d'une allergie, la composition comprenant :
un antihistaminique comprenant au moins l'une parmi la chlorphéniramine et la diphénhydramine, l'antihistaminique comprenant une concentration entre environ 0,25 % et environ 4 % en masse ;
du xylitol à une concentration entre environ 10 % et environ 15 % en masse ;
du propionate de fluticasone à une concentration entre environ 0,03 % et environ 1,0 % en masse ; et
de l'aloe vera à une concentration entre environ 0,02 % et environ 3,0 % en masse.

4. Composition de pulvérisation nasale selon la revendication **3,** dans laquelle l'antihistaminique comprend de la chlorphéniramine.

5. Composition de pulvérisation nasale selon la revendication **4,** dans laquelle la chlorphéniramine est présente dans la composition à une concentration entre environ 0,4 % et environ 1,0 % en masse.

6. Composition de pulvérisation nasale selon la revendication 3, dans laquelle l'aloe vera est présente dans la composition à une concentration entre environ 0,05 % et environ 2,0 % en masse.
